# EUROPEAN PATENT APPLICATION

(11) **EP 2 919 004 A1**
(43) Date of publication of application: **16.09.2015**
(21) Application number: 15000444.8
(22) Date of filing: 13.02.2015
(51) Int. Cl.: G01N 30/90, C12Q 1/04, B01J 20/286, B01J 20/287, B01J 20/32, B01J 20/10

(54) **Bioassays on modified TLC/HPTLC plates**

(30) Priority: 14.03.2014 EP 14000966
(71) Applicant: Merck Patent GmbH, 64293 Darmstadt (DE)
(72) Inventor: Klingelhoefer, Ines, 35415 Pohlheim (DE); Morlock, Gertrud, 70599 Stuttgart (DE)

(57) **Abstract**

This invention relates to a method for perfoming bioassays directly on TLC plates. By using modified wettable silica based TLC plates the quantitative bioautography is possible directly on the TLC plate.

## Description

### Field of Invention

This invention relates to a method for perfoming bioassays directly on TLC plates. By using modified wettable silica gel based TLC plates the quantitative bioautography is possible directly on the TLC plate.

### Background

The principle of TLC (thin-layer chromatography) is known to a person skilled in the art. Typically small volumes of different solutions with mixtures of analytes to be separated are applied to a porous thin-layer chromatography plate. The plate carrier is typically a sheet of glass, plastic, or aluminium foil, which is coated with a thin layer of adsorbent material, usually silica gel, aluminium oxide, or cellulose. This layer of adsorbent is known as the stationary phase.

After the sample has been applied on the plate, a solvent or solvent mixture (known as the mobile phase) is drawn up the plate via capillary action. Because different analytes ascend the TLC plate at different rates, separation is achieved.

For very complex mixtures a 2-dimensional TLC can be used.

TLC is used for many applications where a chermical or physical detection of the analytes is possible. The application of TLC in combination with bioassays in which the detection is achieved via the growth of microorganisms or enzymes is very limited.

One example where such an assay combination would be of high value is food industry.

Over 80 million small substances are registered in Chemical Abstracts Service. With its daily increasing number, the threat of potential (xeno-) contaminants and residues inclusive of their degradation products and metabolites to the global food/feed chain increases. Even excellent multi-methods will not cope with this challenge in the future or can only contribute to a certain extent in identifying noxious matter. Additionally, food is complex in matrix and less is known about foodborne noxious matter or effects on health and wellness. Consequently, effect-directed analysis (EDA) directly combined with chromatography will increasingly attract attention in the field of food science with regard to functional food, food safety, foodborne diseases and wellness, as it can fill this lack in comprehensive information and contribute answers to current contradictory analytical results.

Up to now, the traditional combination of planar chromatography with aqueous bioassays (bioautography) has been unconvincingly, due to diffuse, broad spots after long aqueous incubation times on the plate. The bioassays in combination with planar chromatography lack zone resolution to clearly identify and quantify the compounds. Consequently, they are mainly used for qualitative profiling.

The combination of planar chromatography with a yeast estrogen screen (YES) bioassay was already demonstrated for the recombinant *Saccharomyces cerevisiae* carrying the DNA sequence of the hER together with the reporter gene lac-Z (M. B. Mueller, C. Dausend, C. Weins and F. H. Frimmel, Chromatographia. 60, 207-211, 2004). EDCs induced the expression of the lac-Z gene, which encoded the enzyme ß-gafactosidase, reacting with 4-methylumbelliferyl-ß-D-galactopyranoside (MUG) to 4-methylumbelliferone (MU; blue fluorescent at UV 366 nm). However, after several hours of plate incubation with the aqueous yeast cell suspension, even bandwise applied samples resulted in diffuse and broad spots. Further method optimization studies remained unsatisfactory. Although it was possible to detect individual standard substances with the so-called planar YES (pYES), using the developed TLC plate as support instead of e.g. a cuvette or microtiterplate, standard mixtures could not be quantified due to the strong zone diffusion effects on the TLC plate.

It would thus be desirable to find an improved method for combining TLC with bioassays.

It has been found that it is possible to substantially improve this combination by using a certain type of TLC plate. Wettable, modified silica based TLC plates have been found to be ideal for performance of bioassays directly on a TLC plate. When performing e.g. an HPTLC-pYES assay with the modified wettable plates even after up to 24 hours of aqueous incubation, the HPTLC-pYES zones remained sharply bounded. This resulted in a reliable, quantitative and sensitive detection of the bioactivity in complex samples. The general applicability of this substantial improvement was demonstrated by transfer of this technology also to the *Bacillus subtilis* bioassay used for screening of antibiotics.

The present invention is therefore directed to a method for performing a bioassay by
a) Performing a TLC separation of one or more samples on a wettable silica gel-based TLC plate which is modified with reversed phase, CN, diol or amino group ligands or combinations thereof.
b) Evaporating the mobile phase
c) Contacting one or more areas of the TLC plate with an aqueous suspension comprising microorganisms and enzymes and optionally incubating the plate with the aqueous suspension
d) Detecting the growth or activity of the microorganisms and enzymes on the plate either directly or by the generation of an indirect signal.

In a preferred embodiment, in step a) a water-wettable TLC plate with RP18 W, diol or CN ligands is used.

In another preferred embodiment, detection in step d) is performed by measuring the colour or fluorescence generated by the microorganisms or enzymes metabolizing a fluorescence or colour generating substrate. But also luminescent microorganisms or enzymes are preferred, generating a direct signal.

In one embodiment, in step c) the pH of the TLC plate is 6.5 or more.

In another preferred embodiment, the thickness of the sorbent layer of the TLC plate is between 100 and 250 µm.

In a preferred embodiment, the aqueous suspension comprises microorganisms.
Fig. 1 shows a time dependence of the MU fluorescence signal intensity for the reduced MUG substrate concentration (0.50 mg/mL). E2 (1.0 - 25.0 pg/band) applied on the HPTLC plate finally released β-galactosidase reacting with the MUG substrate for different periods (15 - 60 min) to form the fluorescent MU signal measured every 15 min (n=2). Further details can be found in Example 2.
Fig. 2. shows dose-response curves (n=4) for 6 EDCs. Measured fourfold on four different plates and days. Further details can be found in Example 3.
Fig. 3. HPTLC-ESI⁻MS of an unknown ECD detected in a sample.

Confirmation of the unknown's identity to be CAPE: standard solution of CAPE recorded (A) without and (B) with fragmentation in comparison to the unknown ECD band in sample P4 recorded without (C) and with (D) fragmentation, revealing the same daughter ions as the CAPE standard solution. Further details can be found in Example 5.

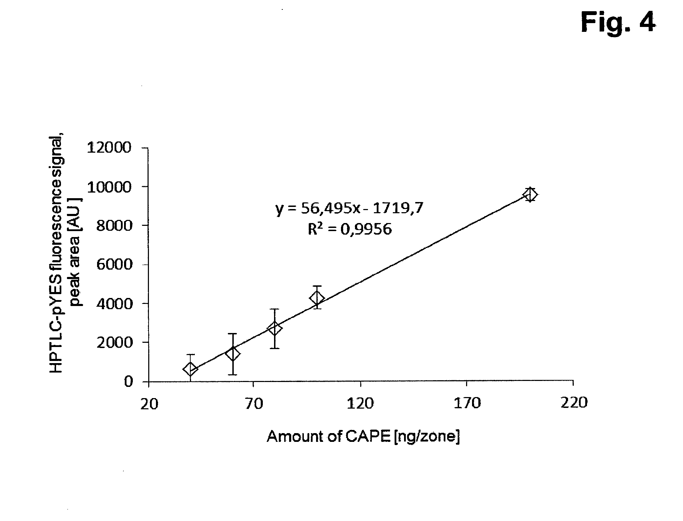

Fig. 4: HPTLC-pYES calibration curve of CAPE. Working range was 40-200 ng/band (n=2) for sample analysis. Further details can be found in Example 6.

TLC plates are known to a person skilled in the art. They typically comprise a support layer made of glass, plastic or a metal foil like aluminum foil and a sorbent layer. The sorbent layer of the plate to be used in the present invention is silica based. That means it is made of SiO₂ which is modified with reversed phase, CN, diol or amino ligands.

TLC means thin layer chromatography. It covers classical TLC as well as HPTLC and any other method using a sorbent plate for chromatographic separation.

Ligands are functionalities which are covalently bonded to the silica sorbent layer. Typically ligands are introduced by reacting the silica sorbent layer with the respective silanes. This is known to a person skilled in the art.

Reversed phase, CN, diol or amino ligands are ligands well known to a person skilled in the art of chromatography.

Reversed phase ligands are typically linear C2 to C18 alkyl ligands.

CN is a ligand carrying at least one CN group. Typically the ligand is an alkyl ligand carrying a CN group.

Diol is a ligand carrying at least two OH groups. Typically the ligand is an alkyl ligand carrying two OH groups.

Amino is a ligand carrying at least one NH₂ group. Typically the ligand is an alkyl ligand carrying one Amino group.

A wettable TLC plate is a plate which when brought in contact with water is wetted by the water. That means the water soakes in the sorbent. This is possible for example with pure silica plates. In contrast to wettable plates, the water applied to non wettable plates forms a droplet on the plate and does not immerse in the sorbent of the plate. A theoretical explanation of wettability is as follows:

Wettablility can be explained with the aid of the adhesive forces between a liquid and solid that cause a liquid drop to spread across the surface. Cohesive forces within the liquid cause the drop to ball up and avoid contact with the surface. The contact angle (θ) is the angle at which the liquid-vapor interface meets the solid-liquid interface. The contact angle is determined by the resultant between adhesive and cohesive forces. As the tendency of a drop to spread out over a flat, solid surface increases, the contact angle decreases. Thus, the contact angle provides an inverse measure of wettability. A contact angle less than 90° (low contact angle) usually indicates that wetting of the surface is very favorable, and the fluid will spread over a large area of the surface. Contact angles greater than 90° (high contact angle) generally means that wetting of the surface is unfavorable so the fluid will minimize contact with the surface and form a compact liquid droplet.

For water, a wettable surface may also be termed hydrophilic and a non-wettable surface hydrophobic. Superhydrophobic surfaces have contact angles greater than 150°, showing almost no contact between the liquid drop and the surface.

A bioassays according to the present invention is any assay in which living microorganisms or enzymes are used to detect a certain analyte. Typically the growth or the metabolism of the microorganisms or enzymes leads to a detectable signal. The signal can e.g. be the growth or the inhibition of the growth of the microorganisms or activity or inhibition of activity of enzymes or a detectable signal like a colour change or a fluorescence resulting from a substrate which is metabolized by the microorganisms or enzymes. Suitable substrates are for example dehydrogenase activity detecting substrates like tetrazolium salts. The dehydrogenase of living microorganisms converts tetrazolioum salts into intensely coloured formazan. In a preferred embodiment, the bioassay is a bioautography. A detailed review about bioautography can be found in Choma et al. Journal of Chromatography A Volume 1218, Issue 19, 13 May 2011, Pages 2684-2691. An examples of bioassays is the p-YES assay which is explained in detail in the Examples. According to the invention, bioassays also mean enzyme-based biochemical reactions. Also such biochemical reactions, e.g. based on tyrosinase oxidase, xanthine oxidase, (choline)esterase or α/ß-glucosidase, are preferred as these biochemical reactions require an aqueous incubation on the TLC plate. These enzymes also react with a substrate to generate a direct or indirect detection signal. Hence, also biochemical reactions are included in the understanding of the term bioassay.

Microorganisms are preferably yeasts, bacteria, algae and funghi. Examples are *Aliivibrio fischeri, Bacillus subtilis, Staphylococcus epidermidis, Corynebacterium xerosis, Aspergillus niger and Saccharomyces cerevisiae lines.* By the present invention, the traditional bioautography workflow is substantially improved by the finding that also modified wettable silica plates can be used. This fundamental improvement led to clear compound assignments and reliable quantitation for the first time, aside reproducible low detectabilites.

For the method of the present invention, a classical TLC separation is performed. It can be a TLC, HPTLC or any other planar separation technique. The procedure is known to a person skilled in the art. Typically two or more samples are separated in parallel.

After performance of the TLC separation, the mobile phase is evaporated. Depending on the solvents that have been used as mobile phase, the evaporation can be completely or only partly. Typically it is favorable to remove the mobile phase as completely as possible.

Afterwards, the TLC plate is directly contacted with the aquous suspension comprising the microorganisms or enzymes. The composition of the aquous suspension comprising the microorganisms or enzymes is dependent on the type of bioassay that is to be performed. The suspension typically comprises additional components like nutrients that maintain and/or support the growth of the microorganisms or activity of the enzymes as well as buffers and substrates for detection.

An aqueous suspension is any suspension whose liquid part comprises more than 90% of water. The liquid part might thus comprise up to 10% of an organic solvent like a water-miscible alcohol.

The contacting of the TLC plate with the aqueous suspension is typically done by immersing the whole or parts of the TLC plate in the aqueous suspension. But it is also possible to apply the aqueous suspension onto the TLC plate. Depending on the bioassay the TLC plate in contact with the aqueous suspension is typically incubated for 10 minutes to several hours or days. Incubation can be performed at room temperature or at any other temperature suitable for the microorganisms.

It has been found that despite long aqueous incubation times (investigated for up to 24 h) and subsequent visualization via a fluorescence substrate reaction, narrow and sharp bioactive high-performance thin-layer chromatography (HPTLC) zones were obtained, allowing reliable quantitation.

Detection can then be performed by any known method. Typically detection is based on measuring a direct signal like bioluminescence or indirect signal like fluorescence or a colour.

It has been found that the results of the method can be further improved if the TLC plate has a pH of 6.5 or more. In a preferred embodiment it has a pH between 6.8 and 7.5. If necessary, the pH of the plate can be altered by incubating the plate with a buffer solution prior to the application of the aqueous suspension comprising the microorganisms. However, the pH adjustment strongly depends on the microorganisms or enzymes used, as also strongly acidophilic or alkaliphilic or other extremophilic microorganisms or enzymes exist.

Examples of suitable wettable modified silica based TLC plates are HPTLC Silica gel 60 CN F254s, HPTLC Silica gel 60 DIOL F254s, HPTLC Silica gel 60 NH₂ F254s, HPTLC Silica gel 60 RP-18 W F254s plates of Merck KGaA, Germany.

Standard reversed phase TLC plates are typically not wettable but since several decades it is possible to generate wettable RP plates. Such plates can be produced e.g. by reducing the number/occupancy rate of RP ligands on the silica plate, by using certain binders which improve wettability of the plate or by reacting the plate not only with RP ligands but additionally with hydrophilic or middle polar ligands, or in combination.

It has been found that RP-W, diol and CN plates are especially suitable modified wettable silica based TLC plates. Suitable wettable RP plates are e.g. RP-18 plates which have a carbon content below 15 % carbon. Suitable RP 18 plates are also plates which have a surface coverage of 1 µmol/m² or less. Such wettable modified plated show on the one hand a sufficient water wettablility so that the bioassay can be performed on the plate. On the other hand, despite the fact that the plate surface had to be still water wettable, it is sufficiently lipophilic to reduce zone diffusion caused by aqueous incubation and allow water to have no elution power (solvent strength). On such lipophilic surfaces, the analytes have higher adsorption energy to the adsorbent than to the aqueous bioassay medium. Also, functionality (layer modification, binder material) was demonstrated to have an influence on bioassay detection, especially on detectability. It was most remarkable that not only on the cyano and diol phases the detectability was very low, but also on the HPTLC silica gel 60 WRF_{254S} layers. The latter showed the best detectability (down to 250 fg/zone), although the zone shape was more diffuse. The eased dissolution of the analyte in the aqueous bioassay and availability for the bioassay reactions, contribute to the low detectability, however, leading to more diffuse zones. With regard to detectability, the diol phase was found to be the second best, although tending to be more diffuse, if compared to the RP-18 W or cyano phases. Comparing latter phases, the cyano plate showed a comparable to slightly better detectability than the RP-18 W plate at comparable band sharpness. To conclude, the arguments referred to band sharpness (zone resolution) as well as detectability (analyte availability to the bioassay). Based in this plate study, the HPTLC plates RP-18 W and CN plates showed especially good properties with regard to sharpness and zone resolution. With regard to low detectability, for example, HPTLC silica gel 60 WRF_{254S} and the diol phases showed good results. Thus a tailor-made layer combination can even improve the discovered potential.

The thickness of the sorbent layer is typically between 50 and 250 µm, preferably between 100 and 250 µm. HPTLC plates typically have a layer thickness around 50 to 200 µm, TLC plates are slightly thicker. The choice of the layer thickness typically depends on the TLC separation to be performed. Concerning the stability of the plate when being incubated with the aqueous suspension it can be favourable to have a layer thickness of more than 100 µm as a thicker layer is typically more stable against cracking.

The combination of chromatography with bioassays can solve the matrix problems associated with sum parameter assays for complex samples. In HPLC/MS and GC/MS, matrix effects are a major source of uncertainty in quantitative trace analysis. Also in this field of quantitative trace analysis, this substantially improved bioautography workflow can contribute due to its specificity (effect-directed detection), good detectability (down to fg/zone levels) and reduced sample preparation (taking raw sample extracts), as demonstrated for the analysis of EDCs in complex samples like spices and propolis by the HPTLC-pYES. Due to the parallel chromatography, up to 20 samples can simultaneously be screened within 4 hours (calculated as 11 min/sample). If compared to Petri dish, cuvette or microtiter plate assays, this strategy allows the specific direct link to a single bioactive compound at very low detectabilites. Without enrichment steps, the sub-ng/kg range can be reached for rectangular applications. All relevant compounds are individually targeted in a complex sample, such as (xeno-, process-)contaminants, residues, foodborne noxious matters, degradation products, and metabolites. Additionally, the mass spectra of discovered, unknown ECDs can be recorded from the same chromatographic run, if the sample was applied twice and cut (one section for bioassay, second for MS). Depending on the bioassays, also the recording of mass spectra directly from the bioactive zone (inclusive of the bioassay medium) is possible. Or the integration of an additional short monolithic column in the TLC-MS Interface outlet line to the MS, allowing orthogonal normal phase (NP)-HPTLC-RP-HPLC-MS. HPTLC in combination with bioassays, derivatization reagents, spectroscopic and high-resolution mass spectrometric detections offers a fast bioactivity profiling and the direct link to the bioactive compounds of interest in complex raw samples. All these tools, inclusive of structure elucidating methods like NMR, FTIR, SERS and HRMS, can be performed at the analytical level directly from the bioactive zone of interest on the HPTLC plate, optionally via the versatile TLC-MS Interface. HPTLC exists in the shade of the powerful HPLC, however, it has been proven that hyphenated HPTLC deserves its place in modern analytical laboratories. The wealth of information gained from using this technique has been key to progress on many of our projects. The future potential for HPTLC in combination with genotoxic or mutatoxic cell test systems is evident and new avenues are opened.

The present invention thus provides a simple but effective way to improve bioautography and allows for the quantitative detection of the outcome of a bioassay directly on a TLC plate or any other planar separation medium.

This invention is further illustrated by the following examples which should not be construed as limiting. The contents of all references, patents and published patent applications cited throughout this application as well as the Figures, and of corresponding EP application EP 14000966.3, filed March 14, 2014, are incorporated herein by reference.

### Examples

### Materials and Methods:

**Propolis extracts.** Liquid samples (P1 - P5) were used directly or diluted 1:10 with ethanol, whereas solid samples (P6 and P7) were extracted with 1 mL ethanol.
   - KAPI Propolis (30% in ethanol), P 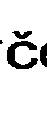 elarska kuća Kova 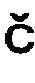 ević, Belgrad, Serbia (P1).
   - 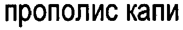 (30% solution), 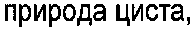 Serbia (P2).
   - KAPI Propolis extra (25% in ethanol), Sinefarm, Belgrad, Serbia (P3).
   - Propolis Ø (62% in ethanol, m/m), Hansosan, Garbsen, Germany (P4).
   - Propolis (drops), allcura Naturheilmittel, Wertheim, Germany (P5).
   - Bakanasan Propolis capsules (250 mg/capsule), Börner, Bremen, Germany (P6); content of one capsule was extracted.
   - Sanhelios Aagaard Propolis throat lozenges (30 mg/suck lozenges), Börner (P7); lozenges were pestled and 100 mg of the crushed material were extracted.

**Spice extracts.** Spice samples (0.5 g; Martin Baur Group, Vestenbergsgreuth, Germany) were pestled and extracted with 5 mL methanol - ethanol 1:1, v/v. The suspension was heated to 60°C for 30 min, followed by 20 min ultrasonification and centrifugation at 12500 rpm for 5 min. The supernatant was stored at -20°C. For removal of chlorophylls, the supernatant (0.5 mL) was diluted with bidestilled water (0.25 mL) and vortexed. Petrolether (0.5 mL) was added, this 3:2 mixture was vortexed and centrifuged at 12500 rpm for 5 min. The resulting upper and lower phase were investigated separately. Bioactive compounds were detected in the lower polar phase, as depicted.

**Standard solutions.** For the direct bioautography, stock solutions of estrone (E1, 95%), estriol (E3, 95%), both Cayman Chemical Company, MI, USA, 17ß-estradiol (E2, 98.5%), 17α-ethinylestradiol (EE2, 98%), both Dr. Ehrenstorfer, Augsburg, Germany, bisphenol A (BPA, 97%) and 4-n-Nonylphenol (NP, 98%), both Alfa Aesar, Karlsruhe, Germany, were prepared at concentrations of 2 mg/mL with ethanol (for E2, E3, EE2 and BPA) or methanol (for E1 and NP), both Roth, Karlsruhe, Germany. Caffeic acid phenethyl ester (CAPE, ≥99%, obtained from Roth) was dissolved in methanol (0.1 mg/mL). All standard solutions were stored at -20°C until use.

**pH measurement of HPTLC plates.** A plate strip was dipped into each a lac-Z buffer (pH 7) and citrate buffer (pH 12) and dried. About 3 cm² of silica gel were converted from the glass surface into a beaker and suspended in 10 mL H₂O. The pH value of this suspension was measured.

**HPTLC.** Standard and sample solutions (0.1-10 µL) were applied on the HPTLC plate RP-18 W (Merck Millipore, Darmstadt, Germany) as 6.5 mm bands by the Automatic TLC Sampler 4 (ATS4; CAMAG, Muttenz, Switzerland). Other TLC/HPTLC plates were investigated (Table S1, Merck Millipore). The standard mixture was applied by overspaying of the individual standard solutions, which allowed flexibility for individual mixtures and volumes. For determination of the recovery rate, 0.1 µL P1 sample was sprayed on the plate and oversprayed with 0.1 µL - 2 µL CAPE standard solution. After sample application, the start zones were dried for 1 min using a hair dryer. Development was performed in a Twin Trough Chamber (20 x 10 cm, CAMAG), using n-hexane - toluene - ethyl acetate 4:1.5:1, v/v/v (all from ROTH) up to a migration distance of 70 mm. After development, the plate was dried with cold air using a hair dryer for 2 min to remove the residual solvent. The developed plate was documented at 366 nm illumination using the TLC Visualizer (CAMAG).

**Cell cultivation.** *Saccharomyces cerevisiae* BJ3505 (protease-deficient, MATα, PEP4: HIS3, Prb1Δ1.6R, HIS3Δ200, lys2-208, trp1Δ101, ura3-52) generated by McDonnell *et al.* (61, 62) was used for the pYES-assay. All experimental studies were based on stock of the recombinant estrogen sensitive yeast cells stored in 30% glycerol supplemented growth media, consisting of D-glucose 1 g/L, yeast nitrogen base 6.8 g/L, both Sigma Aldrich, Steinheim, Germany, and amino acid components according to Routledge and Sumpter (50), at -80 °C. From the stock, cells were streaked on an agar plate with growth medium (30°C, two days) to supply colonies for further suspension cultivation. Shaking suspension cultures were inoculated in 20 mL growth medium and cultured at 30 °C at 100 rpm for 18 h. Prior to the assay, cell concentration and viability determination was performed using a hemocytometer and trypan blue (0.4% Sigma Aldrich) staining. For the pYES, the cell number was adjusted to 2.5 x 10⁸ cells in 50 mL with 150 µM CuSO₄ supplemented growth medium. To remain constant cell quality within the exponential growth phase for further studies, cells were split daily by 1:10 dilution.

**HPTLC-pYES.** As the HPTLC was developed and cells were prepared, the pYES assay was started. The plate was automatically immersed into the cell suspension using the TLC Immersion Device (CAMAG) with a speed of 3.5 cm/s and an immersion time of 5 s. Thus, the cell suspension was homogeneously transferred onto the plate surface. The wettened plate was incubated in a horizontal position in a plastic box under almost 100% humidified air at 30 °C for 3 h. Thereafter, the plate was dried with cold air using a hair dryer for 2 min. For determination of the resulting ß-galactosidase, the plate was treated with the substrate 4-methylumbelliferyl-ß-D-galactopyranoside (MUG, Roth). ß-galactosidase acts on the substrate MUG to produce 4-methylumbelliferone (MU), detected as blue fluorescence at pH>7. MUG was dissolved in DMSO (20 mg/mL, Roth) and added to the reaction buffer (citric acid 6 g/L, disodium hydrogen phosphate 10 g/L, pH 12, Merck) to a final concentration of 0.5 mg/mL. The dried plate was immersed in the substrate solution according to the previous speed and immersion time. A second incubation period of 60 min at 37°C followed. The substrate reaction was terminated by immersion of the plate into a solution of glycine - NaOH (0.1 M, Roth), pH°12. An increase in the fluorescence signal could be achieved by a second and third immersion with the glycine-NaOH solution. Fluorescence measurement at 366/>400 nm was performed with the mercury lamp using the TLC Scanner 3 (CAMAG). Images were taken at 366 nm illumination using the Reprostar 3 documentation system (CAMAG).

**Long-term (24 h) cell cultivation on the HPTLC plate.** One yeast cell colony on the agar plate was suspended in the culture medium (20 mL, as described) and pre-cultivated for 24 h in the incubator at 30°C. This culture suspension was diluted 1:10 with the medium, in which the developed plate was immersed as described. The plate was incubated in a horizontal position in a plastic box under almost 100% humidified air at 30 °C for 24 h.

**HPTLC-MS.** Developed unkown bioactive zones in the propolis samples were analyzed using a single quadrupole mass spectrometer (CMS, Advion, Harlow, UK). HPTLC was coupled to ESI-MS via the TLC-MS Interface. For elution, the oval elution head (4 mm x 2 mm) was employed using methanol - ammonium formate buffer (10 mM, pH 4, 98:2, v/v) at a flow rate of 0.1 mL/min. Full scan mass spectra *(m*/*z* 100-450) were recorded in the negative ionization mode (ESI⁻) using as capillary temperature 250°C, voltage 170 V, source voltage offset 25 V (for fragmentation 35 V), source voltage span 25, source gas temperature 250°C, and as ESI voltage 3 kV.

### 1. Cell growth on TLC plates (pYES after 24 h aqueous cultivation and incubation on the plate)

A yeast cell colony on the agar plate was suspended in the medium and pre-cultivated for one day. The protocol for further cell cultivation in the broth was transferred onto the HPTLC plate followed by plate incubation. This long-term combined cultivation/incubation lasted for 24 h and showed that the yeast cells are able to grow on the plate. The sharpness of the zones even after one day aqueous incubation on the plate confirmed this substantial improvement of the bioautography workflow. Additionally, it was a first proof-of-principle of successful cell growth directly on the plate. Zone sharpness was demonstrated after a one-day aqueous incubation on the plate.

Six different EDCs were applied in descending concentrations on the plate; after pre-cultivation, yeast cells grew on the HPTLC plate and reacted with the EDCs; sharp-bounded, blue fluorescent MU zones were obtained even after 24 h of aqueous cell growth and incubation, both combined on the plate.

### 2. Optimization of the pYES workflow.

To adapt the pYES assay to the lipophilic, but wettable RP-18 W plate surface, a number of assay parameters were investigated. Special focus was laid on the optimization of the enzyme kinetics, including of substrate concentration, response time and pH value. These three tests were performed for merely applied bands (not developed), but the outcome was comparable to chromatographed zones. The reaction of the released ß-galactosidase with the MUG substrate was studied on the example of E2. Up to a concentration of 0.66 mg/mL MUG in citrate buffer a continuous increase of the fluorescent MU product was observed, then a substrate inhibition was evident.

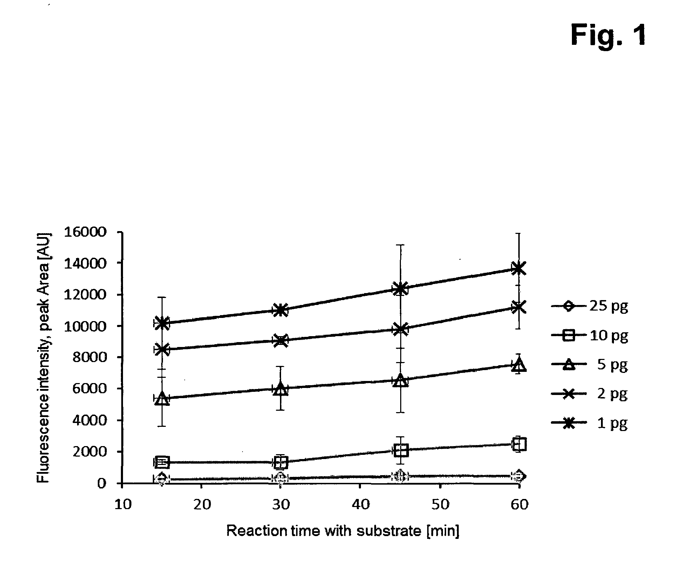

For the previously determined optimal substrate concentration (0.66 mg/mL MUG in citrate buffer), the optimal incubation period was determined next. The MU fluorescence was scanned in 10 min intervals. An increase in the MU fluorescence signal was observed up to 40 min, which decreased rapidly then (Fig. S3). At the 40 min incubation time, the MU fluorescence signal was stable (about ± 5 %) over 1 h, whereas the other incubation periods led to a decrease in the fluorescence signal. The substrate concentration was reduced to 0.50 mg/mL MUG in citrate buffer to improve the reproducibility, which was assumedly impaired due to the high substrate load on the plate. The plate incubation time was investigated at 15 min intervals. Over a period of 60 min an increase in the fluorescence signal was observed and the plate incubation time was extended to 1 h (Fig. 1), using the lower MUG substrate concentration (0.50 mg/mL). The benefit was the absence of the previously observed fluorescence quenching (caused by substrate overload) and a stable fluorescence signal over a wide concentration range.

Further, the pH value of the MUG substrate solution had to be adapted to the lipophilic plate surface and was investigated in the range of pH 6 to 12. The best MU fluorescence signal intensity for E2 was obtained at pH 12. For all YES assays, pH 7 was applied for the enzyme-substrate reaction in the lac-Z buffer. The usually applied lac-Z buffer could not be adjusted to this high pH 12 value, since MgSO₄ precipitated in the solution. Thus, a citrate buffer (54) was preferred. Also, the pH value of the plate surface was analyzed due to the remarkable pH adjustment to pH 12 *(versus* pH 7). A plate strip was dipped each into a lac-Z buffer (pH 7) and citrate buffer (pH 12). The measured pH value was 5.8 to 6.2 for the lac-Z buffer and 7.2 to 7.4 for the citrate buffer. A neutral pH, which was only assured with the citrate buffer, was crucial for an optimal substrate reaction and so for a good detectability. Obviously, the RP-18 W plate, which had an original pH value measured to be 4.8, required a stronger neutralization, if compared to the commonly used silica gel layers (measured to be pH 7.8)

### 3. HPTLC-pYES dose-response curves, LOD, LOQ and working range.

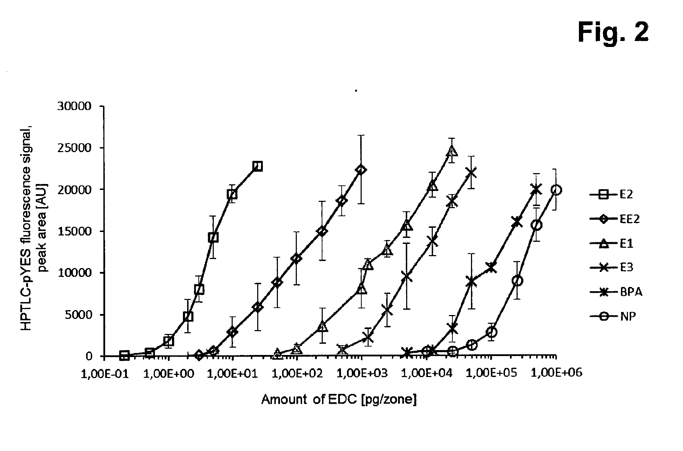

For E2, EE2, E1, E3, BPA and NP, the dose-reponse curves were investigated after their separation on the RP 18W plate (Fig. 2). The half maximal effective concentration (EC50) was calculated for comparison of the receptor affinities. The respective E2 equivalent (E2Eq) was calculated as EC50_{E2}/EC50ₛₐₘₚₗₑ.

The receptor affinity continually decreased in the order E2>EE2>E1>E3>BPA>NP (Table 1). In our study, the EE2 value determined for the modified pYES assay was 0.3, which was the most sensitive and closest to the L-YES assay.

The dose-response curves, performed 4 times on 4 different plates and days, showed a high standard deviation (Fig. 2) and the large fluctuations in the biological test system required continuous calibration of the experiments. E2 showed less deviation if compared to EE2. For the first time, such a detailed study on the E2Eq order was reported for HPTLC-pYES. Owed to the substantially improved zone resolution, a comprehensive comparison of the receptor affinities was possible and conducted in this study.

**Table 1 Performance data of the modified HPTLC-pYES assay**

| Substance (*hR_{F}*) | Structure formula¹ | Maximal working range [pg/zone] | E2Eq | LOD [pg/zone] | LOQ [pg/zone] |
|---|---|---|---|---|---|
| | | R² range (mean R², n=4) | | (n=3) | (n=3) |
| E2 | 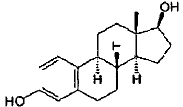 | 0.5 - 50 | 1 | 0.5 | 1 |
| (15) | | 0.929-0.991 (0.96) | | | |
| EE2 | 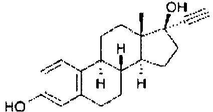 | 2 - 1x10³ | 0.3 | 2 | 5 |
| (24) | | 0.924-0.991 (0.99) | | | |
| E1 | 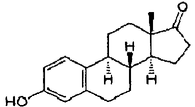 | 25 - 25x10³ | 1.5x10⁻² | 25 | 50 |
| (29) | | 0.930-0.999 (0.98) | | | |
| E3 | 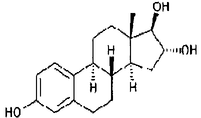 | 5x10² - 50x10³ | 4.2x10⁻³ | n.a. | 500 |
| (2) | | 0.996-0.997 (0.98) | | | |
| BPA | 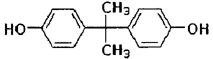 | 62.4x10² - 1x10⁶ | 3.3x10⁻⁴ | 62.5x10² | 12.5x10³ |
| (22) | | 0.960-0.993 (0.97) | | | |
| NP | 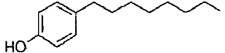 | 25x10³ - 1x10⁶ | 2.3x10⁻⁴ | 25x10³ | 50x10³ |
| (66) | | 0.986-0.991 (0.94) | | | |

The limit of detection (LOD, signal-to-noise ratio 3), the limit of quantification (LOQ, signal-to-noise ratio 10) and the maximal working range for E2, EE2, E1, E3, BPA and NP were determined (Table 1, n=3). LODs ranged down to 0.5 pg/zone for E2. LODs reported for chromatographed zones in literature were in the same range. Despite the sharp-bounded zones and thus high zone resolution, LOD was not that improved, assumedly to the lower availability for the initial binding reaction to the hER (higher adsorption energy to the adsorbent than to the aqueous bioassay). In the detailed previous plate study even better sensitivities were shown on the diol, cyano and silica gel W plates. Nevertheless, the reported LODs are already highly suited for direct EDA of ECDs in complex samples at a physiologically active concentration level. Concentrations down to the sub-ng/kg level can directly be detected, if rectangular application up to 300 µL (e.g. applied within 7 min) is used.

So far, the working range was reported to be between 1.6 and 120 pg/zone for EE2. However in this study, due to the improved zone resolution and the adjusted MUG substrate concentration, the maximal working ranges covered 2 to 3 decades. This extended working range is highly suited for screening of unknown samples and significantly improved the general applicability of the pYES assay.

A major drawback with direct bioautography was reported to be the limited usage of mobile phases for chromatography. Solvents of low volatility such as *n*-butanol as well as acids or bases such as acetic acid, trifluoroacetic acid and ammonia were reported to inhibit the growth of the bacteria, as these cannot be removed completely even after prolonged drying. This drawback was solved in a current study, in which it was demonstrated that even the use of strong acids like hydrochloric acid can be tolerated by cell suspensions. All in all, the good performance data of the substantially modified pYES assay proved its suitability for routine analysis and detection of ECDs.

### 4. Estrogenic properties of commercial propolis samples.

Commercially available propolis fluids were investigated without further sample preparation. The propolis samples as capsule and tablet formulations were extracted with ethanol. All propolis samples were applied two-fold on the HPTLC plate. The complex samples were separated and documented at UV 366 nm. The developed HPTLC plate was subjected to direct bioautography with the pYES assay and again documented at UV 366 nm. Additionally, fluorescence measurement was performed at UV 366/>400 nm. In all propolis samples, three to six bioactive ECD zones were repeatedly detected (bands a - f). The investigated propolis samples consistently showed the bioactive zones (b) and (d), whereas they differed in the zones (a), (c), (e) and (f). Two different propolis types with regard to the discovered EDCs were distinguished, whereby the EDC profiling was not consistently congruent with potential native spectral characteristics. The native fluorescence could also be caused by a co-eluting compound in such complex mixtures.

High sample application volumes or concentrations reduced the zone resolution. For this study, the developed HPTLC plate was cut and one plate section was derivatized by immersion into Neu's reagent followed by dipping in polyethylene glygol (PEG) 4000 solution and drying to be informed on phenolic compounds, as some are known EDCs. The second plate section was subjected to direct bioautography with the pYES assay and also documented at UV 366 nm. In all images, the propolis samples looked overloaded and the clear differentiation of the 3 unknown EDCs in the upper *hR_{F}* range was challenging. However, it clearly revealed weak EDC responses in the lower *hR_{F}* range, like the revealing of the zone a in samples 1-3. Some showed dark zones with fluorescent edges (e.g. zones d or e), which were obtained in case of a very high (too high) fluorescence response. This effect of fluorescence quenching was based on reabsorption or the formation of dimers, and was well-known from HPTLC fluorescence measurements. The fluorescence quenching of the inner darkened zone part was considerably reduced at lower concentrations or reduced sample application volumes. As a negative control, the pYES was performed without yeast cells in the medium. There were no ECD zones detectable

Aside propolis samples, commercially available spices, which are employed in food industry, were screened for EDCs. EDCs were discovered in parsley, oregano, dill and cumin. The EDC zones detected by HPTLC-pYES have to be compared with the native blue fluorescent ones of the merely developed plate, both documented at UV 366 nm. The discovered EDC zone had a characteristic light blue fluorescent hue and its fluorescence intensity increased, if compared to the natively bluish fluorescent zone. Hence, this image comparison between natively bluish fluorescent zone *versus* bluish fluorescent pYES zone is crucial for proper interpretation. Optionally, other substrates than MUG reacting to a visual color can be employed for the pYES assay, e.g. chlorophenolred-ß-D-galactopyranoside.

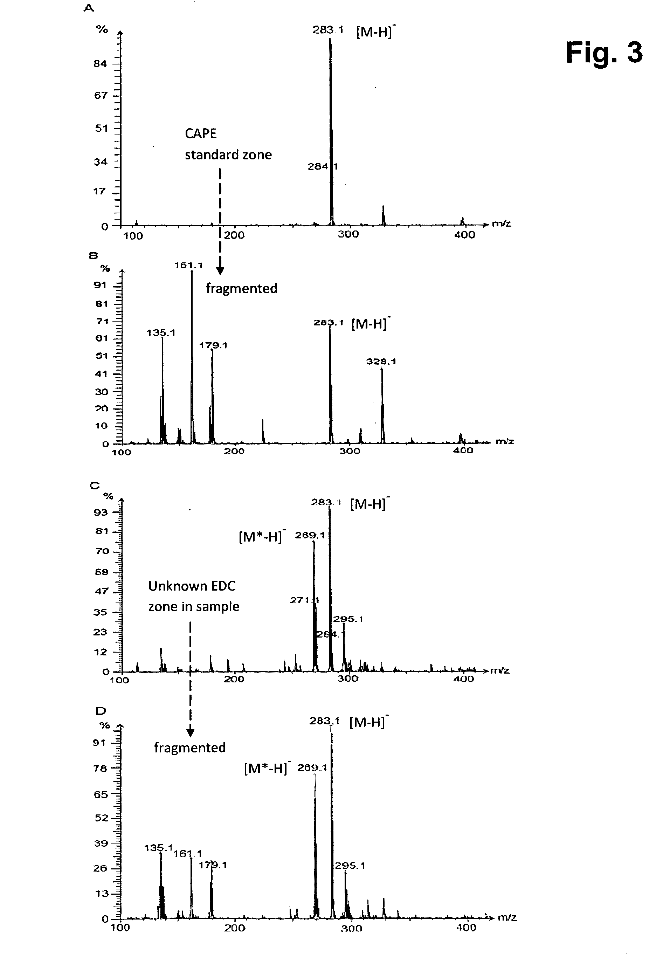

HPTLC offers the possibility to identify unknown zones by overlapped application of the sample with the standard solution. Thus, the standard components migrate partially in the sample matrix and any retention (*hR_{F}* value) shift caused by matrix can be discovered. Matrix effects can thus be recognized or verified. The caffeic acid-phenethylester (CAPE) standard solution partially migrated in the propolis sample. The unknown bioactive EDC zone in the propolis sample was confirmed to be CAPE with regard to its chromatographic performance. For further confirmation, mass spectra were recorded.
**5. Confirmation of unknown EDCs by HPTLC-MS.** Exemplarily, the unknown bioactive zone in the propolis sample, preliminarily assigned as CAPE based on chromatographic and spectral characteristics, was verified through HPTLC-ESI-MS via the elution-head based TLC-MS Interface (Fig. 3). In the negative ionization mode, the CAPE standard zone showed the deprotonated molecule at *m*/*z* 283 [M-H]⁻ (Fig. 3 A) with a specific fragmentation pattern at *m*/*z* 179, 161 and 135 for higher voltages applied (Fig. 3 B). The fragmentation pattern was investigated as a high-resolution MS (HRMS) was not available. The unknown bioactive zone in the propolis sample P4 showed also a mass signal at *m*/*z* 283, confirming the deprotonated CAPE molecule [M-H]⁻ in the sample zone (Fig. 3 C).

However, a further mass signal was observed at *m*/*z* 269 [M*-H]⁻, which fit to the deprotonated molecule of benzyl caffeate. This mass signal was assigned to the band above zone b and appeared in the CAPE mass spectrum due to partial co-elution with the upper adjacent band, caused by lack in resolution and proper elution head positioning. The obtained fragmentation pattern of the propolis sample P4 was comparable to the CAPE standard solution (Fig. 3 D). This confirmation by MS was repeated three-fold. All mass signals obtained were consistent with reported mass signals found in propolis (Fig. 4). With the pYES the phytoestrogens apigenin, chrysin, quercetin and luteolin also showed an estrogenic/bioactive effect. These phytoestrogens were also found in German propolis samples, and their standard solutions were also applied, but these phytoestrogens could not be detected as blue fluorescent EDC zone in the analyzed propolis samples. One reason might be a too low phytoestrogen concentration (below LOD of the applied pYES assay). Another important aspect is the different receptor affinity of these substances to hER. Most of the phytoestrogens have a higher affinity to hERß. The affinity to this receptor was higher by the factor of 20 - 100 if compared to hERα. Hence, the recording of HRMS mass spectra would be helpful.
**6. Quantification of CAPE in propolis samples.** The accuracy of the bioassay was investigated via the standard addition method. CAPE standard zones between 10 and 150 ng/band were oversprayed with propolis samples. Thus, the sample tracks spiked with the CAPE standard were developed and detected with the pYES. The mean recovery of CAPE calculated as standard addition was 95.4 % *(%RSD* 15.2%; n=2) determined at 7 different concentrations ranged 10 and 150 ng/band. A five point calibration was performed to determine the amount of CAPE in propolis samples (Fig. 4).

The content of CAPE was investigated in 7 commercially available propolis samples. The calculated CAPE content ranged 710 - 2387 µg/g, referred to the propolis weight (Table 2, n=2). If compared to literature, the CAPE content in propolis from different geographical regions was determined to be between 1112 and 2525 µg/g by HPLC-MS/MS. Our quantitative results obtained with the modified HPTLC-pYES assay were in accordance with the results obtained by HPLC-MS/MS. For the first time, reliable and verified quantitative results were obtained for HPTLC-direct bioautography. This proved the capabilities for quantitation of the substantially improved HPTLC-pYES assay.

**Table 2. Quantitation of CAPE found in 7 propolis samples by HPTLC-pYES**

| Propolis sample | CAPE content in sample [µg/mL] | CAPE content [µg/g] referred to propolis weight (n=2) |
|---|---|---|
| P1 (30 %) | 481 | 2028 |
| P2 (30 %) | 476 | 2009 |
| P3 (25 %) | 471 | 2387 |
| P4 (62 %) | 348 | 710 |
| P5 (not specified) | 380 | 380³ |
| P6 (250 mg/capsule) | 359¹ | 1435 |
| P7 (30 mg/lozenge) | 22² | 1089 |
| ¹µg/capsule, ²µg/pastille, ³µg/mL | | |

The improvement achieved due to the combination of knowledge can also be transferred to other bioassays. Exemplarily, the general applicability of the substantially improved workflow was demonstrated for the bioassay *Bacillus subtilis.* Also for this bioassay with several hours of aqueous plate incubation time, sharp-bounded bioactive zones were obtained in a botanical sample showing antibiotic activity. As the threat through resistant antimicrobials is increasing and infections are difficult to control, the screening of plant or marine sources, producing an arsenal of secondary metabolites, for new antibiotics is of high priority. This streamlined HPTLC-EDA approach will be an attractive tool for analysts.

## Claims

1. A method for performing a bioassay by
a) Performing a thin layer chromatography separation of one or more samples on a wettable silica-based TLC plate which is modified with reversed phase, CN, diol or amino ligands or a combination thereof
b) Evaporating the mobile phase
c) Contacting one or more areas of the TLC plate with an aqueous suspension comprising microorganisms or enzymes and incubating the plate with the aqueous suspension
d) Detecting the growth of the microorganisms or enzymes on the plate either directly or by the generation of an indirect signal.

2. Method according to claim 1 **characterized in that** in step a) a TLC plate with RP-18 W or CN ligands is used.

3. Method according to claim 1 or 2, **characterized in that** detection in step d) is performed by measuring the colour or fluorescence generated by the microorganisms or enzymes metabolizing a fluorescence or colour generating substrate.

4. Method according to one or more of claims 1 to 3, **characterized in that** in step c) the pH of the TLC plate is 6.5 or more.

5. Method according to one or more of claims 1 to 4, **characterized in that** the thickness of the sorbent layer of the TLC plate is between 100 and 250 µm.

6. Method according to one or more of claims 1 to 5, **characterized in that** the aqueous suspension comprises microorganisms.
